# EUROPEAN PATENT SPECIFICATION

(11) **EP 1 343 526 B1**
(45) Date of publication and mention of the grant of the patent: **21.06.2006**
(21) Application number: 01271227.9
(22) Date of filing: 14.12.2001
(51) Int. Cl.: A61K 39/385, A61K 47/48, A61P 25/34

(54) **Vaccine for the treatment of nicotine addiction**
Impfstoff zur Behandung von Nikotinabhängigkeit
Vaccin pour traiter le tabagisme

(30) Priority: 20.12.2000 GB 0031079
(43) Date of publication of application: 17.09.2003
(73) Proprietor: SMITHKLINE BEECHAM PLC, Brentford, Middlesex TW8 9GS (GB)
(72) Inventor: GRATTAN, Timothy, James, Weybridge, Surrey KT13 0DE (GB)
(74) Representative: Morris, Miriam Elizabeth
(86) International application number: PCT/GB2001/005570
(87) International publication number: WO 2002/049667

(56) References cited:
- WO-A-00/32229
- WO-A-98/14216
- WO-A-99/13907
- WO-A-99/61054

## Description

The present invention relates to novel vaccines and pharmaceutical preparations for the treatment and prevention of nicotine addiction. In particular it relates to vaccines comprising one of the metabolites of nicotine, or pharmaceutical preparations comprising ligands capable of binding to a metabolite of nicotine. The vaccines of the present invention are capable of inducing antibodies which abrogate the effects of the metabolite of nicotine in the body, thereby facilitating smoking control. Also provided by the present invention are novel immunogens comprising a metabolite of nicotine and their use in the formulation of vaccines for smoking control. Methods of treatment of individuals wanting to reduce or quit smoking by administration of the vaccines or pharmaceutical preparations of the present invention, are also provided.

Smoking has an adverse effect on health and consequently smokers often try to quit the habit. However, the addictive nature of nicotine and the availability of cigarettes add to the continued dependence on nicotine and the high failure rate of those trying to quit. Withdrawal symptoms are unpleasant and are usually relieved by further smoking.

One of the major causes of failure to quit smoking is the physical addiction to nicotine. Following administration, nicotine stimulates many receptors in the brain, causing release of dopamine, and thereafter nicotine undergoes extensive biotransformation via a number of metabolic pathways (Kyerematen (1991) Drug Metabolism Reviews 23(1&2), 3-41) into many different metabolites, examples of which include cotinine, nicotine 1'-N-oxide, nicotine glucuronide, nornicotine (Nicotine Safety and Toxicity, Benowitz 1998), cotinine N-oxide, norcotinine, trans-3'-hydroxycotinine and cotinine glucuronide.

Cotinine has been shown to be a major metabolite of nicotine and a study by Benowitz, (Clin Pharmacol Ther (1983) 34(5), 604-611) estimated that 86% of systemically absorbed nicotine is metabolized to cotinine in humans. Cotinine has also been shown (Dwoskin et al, The Journal of Pharmacology and Experimental Therapeutics (1999), 288(2), 905-911) to be the most abundant metabolite in rat brain after peripheral nicotine administration. Studies also suggest that cotinine has psychologic activity and that it can antagonise the effects of nicotine *in-vivo* in humans and animals. (Hatsukami et al. Psychopharmacology (1998) 135;141-150).

Many therapies for nicotine addiction have been developed and used, which focus on the activities of nicotine itself. These therapies have either been in the form of nicotine replacement therapy, such as nicotine containing chewing gum or skin patches, or in the abrogation of the effects of nicotine by vaccination as described in WO 98/124216 (Immulogic Pharmaceuticals Inc) and WO 00/33229 (Nabi). In contrast to these references, the current invention involves the targeting of the metabolites of nicotine.

It has been found, surprisingly, that the targeting of the metabolites of nicotine, by immunotherapy, facilitates smoking control. In particular, one aspect of the present invention involves the immunotherapeutic alteration of the biological effects of the metabolites of nicotine which themselves are antagonists of nicotine. The result of this type of immunotherapy in an individual attempting to quit smoking is the reduction, prevention or alteration of smoking withdrawal symptoms, or the alteration of the positive effects of nicotine intake, thereby increasing the chances of success of the individual quitting smoking. The metabolite of nicotine that is particularly preferred within the context of the present invention is cotinine.

Without wishing to be bound by theory, an immune reaction in a subject against a cotinine metabolite can surpisingly reduce the dampening effect the cotinine metabolite has on the nicotine neurological effect, thereby resulting in the subject craving a lesser amount of nicotine.

The present invention, therefore, provides active or passive forms of immunotherapy against the metabolites of nicotine. For example, there is provided a vaccine comprising a metabolite of nicotine, and in particular those vaccines that comprise cotinine, which is used for immunotherapy of nicotine addiction.

Also provided are immunogens for use in the vaccines of the present invention comprising a metabolite of nicotine, conjugated to a carrier molecule that is capable of converting the poorly immunogenic metabolite into an effective immunogen.

Accordingly, there is provided an immunogen comprising a metabolite of nicotine, and a carrier molecule that comprises T-helper epitopes, such as a protein, for use in the immunotherapy of nicotine addiction. Preferred immunogens of the present invention comprise cotinine conjugated to a protein carrier molecule.

For example anti-cotinine antibodies were purchased from American Research Products Inc (ARP), 489 Common St. #B, Belmont, MA, 02178-4455.

Anti-cotinine antibodies are also available from Cortex Biochem Inc., 1933 Davis St., #321, San Leandro, Ca, 94577 and Advanced Biotechnologies Ltd., Mole Business Park 3, #7 Randalls Road, Leatherhead, Surrey, KT22 7B.

There is also provided by the present invention, use of the metabolites of nicotine, and especially cotinine, in the manufacture of a medicament for the treatment of nicotine addiction.

Also provided by the present invention is the passive therapy of nicotine addiction. Accordingly, pharmaceutical preparations are provided for administration which comprise a ligand capable of binding to, and reducing the activity of, a metabolite of nicotine. In this aspect of the present invention the preferred ligands are polyclonal or monoclonal antibodies. Again, the preferred metabolite of nicotine for passive immunotherapy is cotinine.

The term "antibody" herein is used to refer to a molecule having, a useful antigen binding specificity. Those skilled in the art will readily appreciate that this term may also cover polypeptides which are fragments of or derivatives of antibodies yet which can show the same or a closely similar functionality. Such antibody fragments or derivatives are intended to be encompassed by the term antibody as used herein.

Accordingly, in a related aspect of the present invention are ligands capable of binding to, and reducing the activity of cotinine. The immunogens of the present invention may also be used for the generation of monoclonal antibody hybridomas (using known techniques *e.g.* Köhler and Milstein, Nature, 1975, 256, p495), hurrianised monoclonal antibodies or CDR grafted monoclonals, by techniques known in the art. The use of the immunogens described herein in the manufacture of polyclonal or monoclonal antibodies for the treatment of nicotine addiction is also provided by the present invention.

Novel immunogens are provided which comprise the conjugation of a metabolite of nicotine to a T-helper epitope containing protein carrier.

Cotinine is shown by the following structure:

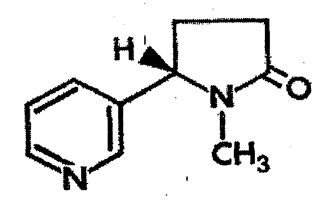

Immunogens of the present invention are made by the linking the protein carrier to any position on the cotinine molecule provided that the linker does not join at the 2' (carbonyl) position.

Particularly preferred cotinine based immunogens of the present invention are shown in the following formula: 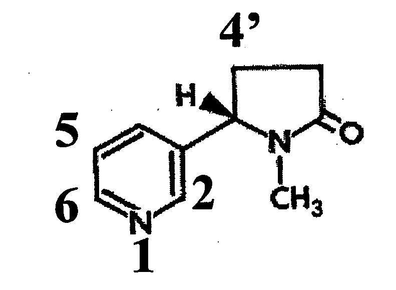 wherein a protein carrier is covalently coupled to the cotinine molecule at any of the positions 1, 2, 5, 6, or 4'.

Specific linkers and the corresponding linker chemistry is shown in WO 98/124216 (Immulogic Pharmaceutical Corporation) WO 00/33229 (Nabi) and WO 99/61054 (Independent Pharmaceutica AB),' all of which are incorporated by reference hereto.

Covalent coupling of the peptide of the metabolite to the immunogenic carrier can be carried out in a manner well known in the art. Thus, for example, for direct covalent coupling it is possible to utilise a carbodiimide, glutaraldehyde or (N-[y-maleimidobutyryloxy]) succinimide ester, utilising common commercially available heterobifunctional linkers such as CDAP and SPDP (using manufacturers instructions). After the coupling reaction, the immunogen can easily be isolated and purified by means of a dialysis method, a gel filtration method, a fractionation method etc.

The types of carriers used in the immunogens of the present invention will be readily known to the man skilled in the art. The function of the carrier is to provide cytokine help in order to help induce an iminune response against the metabolite of nicotine. A non-exhaustive list of carriers which may be used in the present invention include: Keyhole limpet Haemocyanin (KLH), serum albumins such as bovine serum albumin (BSA), inactivated bacterial toxins such as tetanus or diptheria toxins (TT and DT), CRM197 or recombinant fragments thereof (for example, Domain 1 of Fragment C of TT, or the translocation domain of DT), or the purified protein derivative of tuberculin (PPD). Alternatively the nicotine metabolites may be directly conjugated to liposome carriers, which may additionally comprise immunogens capable of providing T-cell help. Preferably the ratio of the number of nicotine metabolites to each carrier is in the order of 1:1 to 20:1, and preferably each carrier should carry between 3-15 nicotine metabolites.

In an embodiment of the invention a preferred carrier is Protein D from *Haemophilus influenzae.* Protein D is an IgD-binding protein from *Haemophilus influenzae* and has been patented by Forsgren (WO 91/18926, granted EP 0 594 610 B 1). In some circumstances, for example in recombinant immunogen expression systems it may be desirable to use fragments of protein D, for example Protein D 1/3^{rd} (comprising the N-terminal 100- 110 amino acids of protein D (GB 9717953.5)).

Alternatively, the carrier molecule may consist of T-helper epitopes, in the absence of other non-helper amino acid sequences (WO 99/67293), or the metabolite of nicotine may be formed into immunogenic matrices (WO 00/33229).

The present invention, therefore, provides the use of the metabolites of nicotine in the manufacture of vaccines for the prophylaxis or therapy of nicotine addiction. Accordingly, the immunogens of the present invention are provided for use in medicine, and in the medical treatment or prophylaxis of nicotine addiction. Accordingly, there is provided a method of treatment of nicotine addiction comprising the administration to a patient suffering from or susceptible to nicotine addiction, of a vaccine or medicament of the present invention.

Vaccines of the present invention, may advantageously also include an adjuvant. Suitable adjuvants for vaccines of the present invention comprise those adjuvants that are capable of enhancing the antibody responses against the metabolite of nicotine. Adjuvants are well known in the art (Vaccine Design - The Subunit and Adjuvant Approach, 1995, Pharmaceutical Biotechnology, Volume 6, Eds. Powell, M.F., and Newman, M.J., Plenum Press, New York and London, ISBN 0-306-44867-X). Preferred adjuvants for use with immunogens of the present invention include aluminium or calcium salts (for example hydroxide or phosphate salts). Other adjuvants include saponin adjuvants such as QS21 (US 5,057,540) and 3D-MPL (GB 2220 211).

The vaccines of the present invention will be generally administered for both priming and boosting doses. It is expected that the boosting doses will be adequately spaced, or preferably given yearly or at such times where the levels of circulating antibody fall below a desired level.

In a further aspect of the present invention there is provided a vaccine as herein described for use in medicine.

The vaccine preparation of the present invention may be used to treat an individual addicted to nicotine, by means of administering said vaccine via systemic or mucosal route. These administrations may include injection via the intramuscular, intraperitoneal, intradermal or subcutaneous routes; or via mucosal administration to the oral/alimentary, respiratory, genitourinary tracts. A route of administration via the transdermal route is, for example by vaccine delivery skin patches.

The amount of protein in each vaccine dose is selected as an amount which induces an immunoprotective response without significant, adverse side effects in typical vaccines. Such amount will vary depending upon which specific immunogen is employed' and how it is presented. Generally, it is expected that each dose will comprise 1-1000 µg of protein, preferably 1-500 µg, preferably 1-100 µg, of which 1 to 50µg is the most preferable range. An optimal amount for a particular vaccine can be ascertained by standard studies involving observation of appropriate immune responses in subjects. Following an initial vaccination, subjects may receive one or several booster immunisations adequately spaced.

Pharmaceutical compositions comprising the ligands, described above, also form an aspect of the present invention. Also provided are the use of the ligands in medicine, and in the manufacture of medicaments for the treatment of nicotine addiction.

Vaccine preparation is generally described in New Trends and Developments in Vaccines, edited by Voller et al., University Park Press, Baltimore, Maryland, U.S.A. 1978. Conjugation of proteins to macromolecules is disclosed by Likhite, U.S. Patent 4,372,945 and by Armor et al., U.S. Patent 4,474,757.

The present invention is illustrated by but not limited to the following examples.

### 1. Preparation of cotinine-protein conjugates

### 1.1 Preparation of N-alkyl pyridyl cotinine conjugate

The production of the following derivatised cotinine molecule was performed as described below.

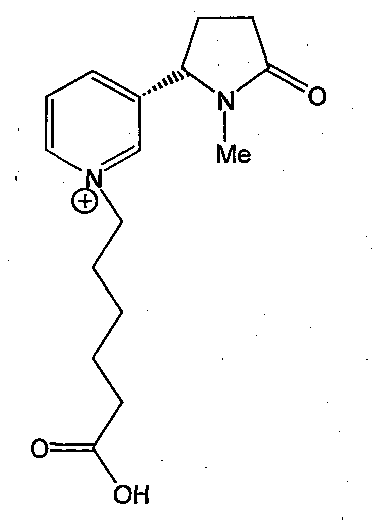

The reaction was conducted under nitrogen, and all glassware was predried in an oven at 150 °C for at least 12 hours prior to use.
(-)-Cotinine (200 mg, 1.1 mmol) was purchased commercially from Toronto Research Chemicals, (www.trc-canada.com) and dissolved in dry methanol (1 cm³; distilled from CaH₂) and cooled to 0 °C in an ice bath and 6-bromohexanoic acid (244 mg, 1.25 mmol) in dry methanol was added dropwise. The solution was then allowed to stir at room temperature for 24 hours. The solvent was then removed *in vacuo,* and water (5 cm³) and CH₂Cl₂ (5 cm³) were then added to the residue. The aqueous phase was washed with CH₂Cl₂ (6 × 5 cm³) (to remove excess bromoacid) until the aqueous phase only showed one baseline product by TLC (methanol). The aqueous solution was then concentrated (freeze drier) to give the *N*-alkylpyridyl cotinine conjugate (90 mg, 27 %) as a colourless oil, δ_{H} (270 MHz, CDCl₃)8.84 (2 H, d, *J* 6.3), 8.47 (1 H, d, *J* 7.9), 8.09 (1 H, dd, *J* 6.3, 7.9), 5.03 (1 H, m), 4.62 (2 H, t, *J* 7.3), 2.72-2.54 (6 H, m), 2.36 (2 H, t, *J* 7.3), 2.09-1.88 (3 H, m), 1.68-1.56 (2 H, m), 1.41-1.29 (2 H, m); *m*/*z* (EI) 291 [M⁺, 3 %], 176 [M-((CH₂)₅CO₂H), 42 %].

Conjugating carboxyl-group-containing molecules to proteins.
Carboxyl groups can be conjugated to amino-groups on proteins using either an active ester intermediate (for example making a N-hydroxysuccinimide ester), or by activating the carboxyl group in situ with carbodiimide.

### Carbodiimide:

When linking to proteins the water soluble carbodiimide is usually used. This is referred to as EDC or EDAC. Other carbodiimides exist, but are generally for organic synthesis.
The hapten is dissolved in water so that there will be a 20-100 fold molar excess over the protein. The pH is adjusted so that the carboxyl group is in the protonated form: this generally requires bringing the pH to below 7, and ideally to about pH 5. At higher pHs the reaction might also work however a higher ratio of hapten to protein may be required in order to get the reaction to work.
A 10-fold molar excess (over the carboxyl group) of soluble carbodiimide 1-ethyl-3-(3-dimethylaminopropyl)carbodiimide (EDC) is then added. This can be added either as a powder, or as a very fresh solution in water. This is left to incubate at room temperature for 3-5 minutes.
The activated hapten-EDC mixture is then added to the protein solution in 100mM phosphate buffer at pH 8.. This buffer system prevents the residual carbodiimide activating carboxyl groups on the protein.
Leave for several hours (5-8) and dialyse against phosphate buffered saline to remove any unreacted derivatised cotinine.

### Hydroxysuccinimide esters

Instead of activating the carboxyl group in situ, for small organic molecules the activation can be done beforehand.
In this case N-hydroxysuccinimide is conjugated to the carboxyl group of the hapten, generally using an organic-soluble carbodiimide such as dicyclohexylcarbodiimide (DCC). The resulting N-hydroxysuccinimide ester will conjugate automatically to amino-groups on proteins:
The requirements are that the activated hapten is soluble in water, and that the protein is at a pH between 7.5 and 8.5.
If the N-hydroxysuccinimide esters are not soluble directly in water, the activated hapten can be dissolved in dimethyl formamide or dimethyl sulphoxide or ethanol, and then a small amount of this organic solution added to the solution of protein in 50 mM phosphate buffer pH 7.5-8. It is important that the organic solution is fairly fresh: the presence of trace amounts of water in these solutions will slowly hydrolyse the active ester. In the subsequent conjugation reaction no more than a 10-20% by volume of organic solvent should be added to the protein solution to prevent the protein from precipitating.
An alternative method is to make the sulfo-N-hydroxysuccinimide ester. In this case there is a sulphate group on the N-hydroxysuccinimide, enhancing the water - solubility.
Generally a 10- or 20-fold molar excess of the activated ester is added to the protein solution in 20-50 mM phosphate buffer pH 7.5-8. A protein concentration of around 1 mg/ml is generally used. Allow the reaction to proceed for 5 hours at least, and then dialyse against phosphate buffered saline to remove any unreacted derivatised cotinine.Dialysis should be at 4°C, and against at least two changes of buffer, and each dialysis should last at least 3 hours.

Other methods of conjugating carboxyl groups to proteins have been described, eg by adding sulfo-N-hydroxysuccinimide to the EDC reaction to help it (in situ formation of a sulfo-NHS active ester intermediate) or using HATU.

### 1.2 Preparation of thiol conjugate of cotinine

The production of the following derivatised cotinine molecule was performed as described below.

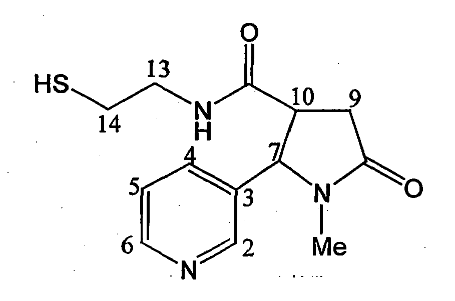

The reaction was conducted under nitrogen, and all glassware was predried in an oven at 150°C for at least 12 hours prior to use.
(±)-*trans*-4-Cotinine carboxylic acid (200 mg, 0.9 mmol), 2-aminoethanethiol.HCl (103 mg, 0.9 mmol) and triethylamine (0.13 cm³, 1.8 mmol) were dissolved in dry DMF (1.5 cm³). The mixture was cooled to 0 °C in an ice bath, and EDCI.HCl (174 mg, 0.9 mmol) was then added. The reaction mixture was warmed to room temperature and stirred for a further 12 hours. The solvent was removed *in vacuo,* and water (5 cm³) and CH₂Cl₂ (5 cm³) were added to the residue. The aqueous phase was extracted with CH₂Cl₂ (4 × 5 cm³), and the combined organic extracts were dried over Na₂SO₄, filtered, and concentrated *in vacuo.* Purification by flash chromatography using 5 % methanol/ dichloromethane as eluent, gave the thiol (136 mg, 54 % yield) as a colourless oil, δ_{H} (400 MHz, CDCl₃) 8.58 (1 H, dd, *J* 2.0, 4.9, H-4), 8.50 (1 H, d, *J* 2.0, H-2), 7.60 (1 H, dt, *J* 2.0, 7.8, H-6), 7.3 (1 H, dd, *J* 4.9, 7.8, H-5), 6.77 (1 H, br t, *J* 5.4, NH), 4.79 (1 H, d, *J* 6.8, H-7), 3.51-3.35 (2 H, m, H-9a, H-9b), 2.92-2.68 (5 H, m, H-10, H-13a, H-13b, H-14a, H-14b), 2.64 (3 H, s, CH₃), 1.32 (1 H, t, *J* 8.3, SH); νₘₐₓ (thin film)/cm⁻¹ 3500-3100 (br NH), 2543 (S-H), 1684 (C=O).

The derivatised cotinine was then conjugated via a thio-ether bond to Ovalbumin as the protein carrier using conventional maleimide chemistry. Heterobifunctional reagents such as GMBS (gamma-maleimidobutyryloxy-succinimide ester) react with amino groups on proteins via the hydroxysuccinimide ester, resulting in maleimide-modified protein, which can then react with thiols.

Manufacturers' recommendations should be followed, but the following conditions were used. GMBS is dissolved in ethanol or dimethylformamide at 1 mg/ml, dissolve protein (OVA) in phosphate buffer or PBS pH 7.5-8. Add a 10-20 fold molar excess of GMBS to the protein. A 10-fold excess tends to give about 5 maleimide groups per ovalbumin molecule. A 20-fold excess gives about 10 maleimide groups per ovalbumin. Allow to react for 2-3 hours. Dialyse against a large volume (11) of phosphate buffer of PBS pH 7.5. After 3-4 hours of dialysis add your thiolated reagent to the modified protein. The thiolated reagent should be added in excess over the maleimide groups, such as a 20-fold excess. Allow to react for > 8hours. Dialyse for several hours to remove unbound hapten.

### 1.3 Preparation of the 6-amino cotinine conjugate

The production of the following derivatised cotinine molecule was performed as described below.

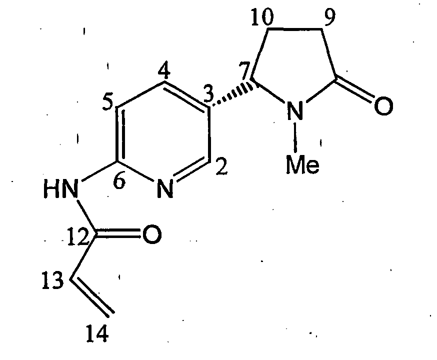

Acrylic acid (0.04 cm³, 0.6 mmol) was added to a solution of 6-amino cotinine (100mg, 0.5 mmol) in toluene (1 cm³) and reflux, and heating was continued for one hour. During this time an oil separated out. The reaction mixture was cooled to room temperature, and the solvent removed *in vacuo.* Water (5 cm³) and dichloromethane (5 cm³) was added, and the aqueous phase was separated extracted with further portions of dichloromethane (3 × 5 cm³). The combined organic extracts were combined and the solvent was removed *in vacuo.* Purification by flash chromatography using 2 % methanol/ dichloromethane as eluent, gave the 6-amino cotinine conjugate (12 mg; 9 %) as a colourless oil, δ_{H} (270 MHz, CDCl₃) 8.66 (1 H, br s, NH), 8.36 (1 H, d, *J* 8.5, H-5), 8.21 (1H, d, *J* 2.2, H-2), 8.21 (1H, dd, *J* 2.2, 8.6, H-4), 6.51 (1H, dd, *J* 1.5, 17.0, H-14a), 6.36 (1 H, dd, *J* 9.8, 17.0, H-13), 5.84 (1 H, dd, *J* 1.5, 9.8, H-14b), 4.54 (1 H, m), 2.70 (3.H, s, CH₃), 2.67-2.46 (3 H, m), 1.97-1.79 (1 H, m).

The acrylamide group of the 6 amino cotinine derivative can be easily converted to a free thiol group by reacting it with thioacetic acid or potassium thioacetate. The free thiol group is then generated by cleaving the thioacteate using either potassium carbonate/methanol or sodium methoxide in methanol. The derivatised cotinine was then conjugated via a thio-ether bond to Ovalbumin as the protein carrier using conventional thiol chemistry.
The heterobifunctional reagent SPDP (N-succinimidyl 3,2-pyridyldithiopropionate) is reacted with the amino groups on the protein, to form protein derivatised with dithiopyridyl. The dithiopyridyl group reacts with thiol groups at pH above 5 forming a disulphide bond (below pH 7 thiols do not react readily with other thiols, so between pH 5 and 7 the reaction favours formation of disulphide bonds between the thiol-bearing hapten and the protein).

### 1.4 Formulation of vaccines

The products of 1.1, 1.2 and 1.3 are formulated into vaccines by adsorbtion onto aluminium hydroxide salt purchased from Superfos.

## Claims

1. A vaccine for use in the treatment of nicotine addiction of individuals wishing to quit smoking, comprising a metabolite of nicotine conjugated to a carrier comprising T-cell epitopes, and an adjuvant.

2. A vaccine according to claim 1, wherein the carrier is a protein.

3. A vaccine according to claims 1 or 2, wherein a linker bridges the metabolite of nicotine and the carrier.

4. A vaccine according to any of claims 1 to 3, wherein the metabolite of nicotine is cotinine.

5. A vaccine according to claim 4, wherein the cotinine is conjugated to the carrier via the 1, 2, 5, 6 or 4' position.

6. A vaccine according to any of claims 1 to 5, for administration via the systemic or mucosal route.

7. Use of a vaccine according to any of claims 1 to 6 for the manufacture of a medicament for the treatment of nicotine addiction.

8. The use of a composition comprising a metabolite of nicotine conjugated to a carrier comprising T cell epitopes for the manufacture of a medicament for the treatment of nicotine addiction of individuals wishing to quit smoking.

9. A process for making a vaccine comprising for the steps of conjugating cotinine via the 1, 2, 5, 6 or 4' position to a carrier comprising T-cell epitopes, optionally via a linker, and adding an adjuvant to the resulting conjugate.

## Patentansprüche

1. Impfstoff zur Verwendung in der Behandlung von Nikotinabhängigkeit von Individuen, die mit dem Rauchen aufhören wollen, der einen Metaboliten von Nikotin, der an einen Träger konjugiert ist, der T-Zell-Epitope umfasst, und einen Hilfsstoff umfasst.

2. Impfstoff gemäss Anspruch 1, worin der Träger ein Protein ist.

3. Impfstoff gemäss Anspruch 1 oder 2, worin ein Linker den Metaboliten von Nikotin und den Träger verbrückt.

4. Impfstoff gemäss einem der Ansprüche 1 bis 3, worin der Metabolit von Nikotin Cotinin ist.

5. Impfstoff gemäss Anspruch 4, worin das Cotinin an den Träger über die 1-, 2-, 5-, 6- oder 4'-Stellung konjugiert ist.

6. Impfstoff gemäss einem der Ansprüche 1 bis 5 zur Verabreichung über den systemischen oder mukosalen Weg.

7. Verwendung eines Impfstoffs gemäss einem der Ansprüche 1 bis 6 zur Herstellung eines Medikaments zur Behandlung von Nikotinabhängigkeit.

8. Verwendung einer Zusammensetzung, die einen Metaboliten von Nikotin umfasst, der an einen Träger konjugiert ist, der T-Zell-Epitope umfasst, zur Herstellung eines Medikaments zur Behandlung von Nikotinabhängigkeit von Individuen, die mit dem Rauchen aufhören wollen.

9. Verfahren zur Herstellung eines Impfstoffs, das die Schritte des Konjugierens von Cotinin über die 1-, 2-, 5-, 6- oder 4'-Stellung mit einem Träger, der T-Zell-Epitope umfasst, gegebenenfalls über einen Linker, und das Hinzugeben eines Hilfsstoffs zum resultierenden Konjugat umfasst.

## Revendications

1. Vaccin destiné à une utilisation dans le traitement de la dépendance à la nicotine des personnes souhaitant s'arrêter de fumer, comprenant un métabolite de la nicotine conjugué à un porteur comprenant des épitopes de cellules T, et un adjuvant.

2. Vaccin selon la revendication 1, dans lequel le porteur est une protéine.

3. Vaccin selon les revendications 1 ou 2, dans lequel un lieur relie le métabolite de la nicotine et le porteur.

4. Vaccin selon l'une quelconque des revendications 1 à 3, dans lequel le métabolite de la nicotine est la cotinine.

5. Vaccin selon la revendication 4, dans lequel la cotinine est conjuguée au porteur via la position 1, 2, 5, 6 ou 4'.

6. Vaccin selon l'une quelconque des revendications 1 à 5, destiné à une administration par voie systémique ou mucosale.

7. Utilisation d'un vaccin selon l'une quelconque des revendications 1 à 6, pour la fabrication d'un médicament destiné au traitement de la dépendance à la nicotine.

8. Utilisation d'une composition comprenant un métabolite de la nicotine conjugué à un porteur comprenant des épitopes de cellules T pour la fabrication d'un médicament destiné au traitement de la dépendance à la nicotine des personnes souhaitant s'arrêter de fumer.

9. Procédé pour fabriquer un vaccin comprenant les étapes de conjugaison de la cotinine via la position 1, 2, 5, 6 ou 4' avec un porteur comprenant des épitopes de cellules T, éventuellement par le biais d'un lieur, et d'ajout d'un adjuvant au conjugué obtenu.
